Europäisches Patentamt

European Patent Office

Office européen des brevets

(11) Veröffentlichungsnummer: **0 290 920 B1**

## (12) EUROPÄISCHE PATENTSCHRIFT

(45) Veröffentlichungstag der Patentschrift: **12.08.92**

(21) Anmeldenummer: **88107064.3**

(22) Anmeldetag: **03.05.88**

(51) Int. Cl.⁵: **B65D 51/30**, B65D 39/00

---

(54) **Behälter für Teststreifen.**

---

(30) Priorität: **13.05.87 DE 3715938**

(43) Veröffentlichungstag der Anmeldung:
**17.11.88 Patentblatt 88/46**

(45) Bekanntmachung des Hinweises auf die
Patenterteilung:
**12.08.92 Patentblatt 92/33**

(84) Benannte Vertragsstaaten:
**AT BE CH DE ES FR GB GR IT LI LU NL SE**

(56) Entgegenhaltungen:
**FR-A- 2 193 741**
**FR-A- 2 260 512**

(73) Patentinhaber: **BOEHRINGER MANNHEIM
GMBH
Sandhofer Strasse 116
W-6800 Mannheim 31(DE)**

(72) Erfinder: **Sacherer, Klaus-Dieter
Westring 17
W-6719 Kirchheim/Weinstrasse(DE)**
Erfinder: **Weiss, Erich
Zwerchgasse 49
W-6800 Mannheim 31(DE)**

(74) Vertreter: **Pfeifer, Hans-Peter, Dr.rer.nat.
Patentanwalt Nowackanlage 15
W-7500 Karlsruhe 1(DE)**

## Beschreibung

Die Erfindung betrifft einen Behälter für Teststreifen, welche zur Analyse von Körperflüssigkeiten dienen. Der Behälter umfaßt einen Behälterkörper mit einer kreisförmigen Entnahmeöffnung und einer zur Achse der Öffnung weisenden Dichtfläche und einen Stopfen zum Verschließen der Entnahmeöffnung. Der Stopfen weist eine Kopfplatte, einen daran angegossenen Hohlzapfen mit einem nach außen weisenden Dichtwulst, eine Trockenmittelzelle innerhalb des Hohlzapfens und ein Abstützelement, durch das der Hohlzapfen radial nach innen abgestützt wird, auf.

Teststreifen werden in zunehmendem Maße für die Analyse von Körperflüssigkeiten, insbesondere Blut- und Urin, eingesetzt. An die Verpackungen solcher Teststreifen werden sehr hohe Anforderungen gestellt, insbesondere weil die Teststreifen außerordentlich empfindlich gegen Feuchtigkeit sind. Um die notwendige Lagerbeständigkeit zu garantieren, müssen Teststreifenbehälter über lange Zeit (mindestens zwei Jahre) praktisch vollständig dicht sein. Diese Dichtigkeit muß auch erhalten bleiben, wenn die Behälter zur Entnahme der einzelnen Teststreifen viele Male (typischerweise 50 mal) geöffnet und wieder geschlossen werden.

Um die beim Öffnen und Schließen unvermeidlich eindringende Feuchtigkeit zu absorbieren, müssen Teststreifenbehälter eine ausreichende Menge Trockenmittel enthalten. Dennoch wird eine möglichst kleine Bauweise angestrebt.

Trotz dieser hohen Anforderungen müssen Teststreifenverpackungen einfach handhabbar, insbesondere leicht zu öffnen und wieder zu verschließen sein.

Über lange Zeit wurden praktisch ausschließlich Teststreifenbehälter verwendet, die aus Glas oder Metall bestanden und mit einem Schraubdek-kel mit Gummidichtung verschlossen waren. Derartige Behälter waren aufwendig in der Herstellung und das Öffnen und Wiederverschließen war schwierig.

In der deutschen Patentschrift 24 06 558 ist ein Behälter und insbesondere ein entsprechender Verschlußstopfen der eingangs bezeichneten Art beschrieben. Das Abstützelement dient dazu, eine ausreichende Abdichtung zwischem dem Dichtwulst und der zugehörigen Dichtfläche sicherzustellen. Zu diesem Zweck ist es axial auf gleicher Höhe wie der Dichtwulst angeordnet, und es wird Wert darauf gelegt, daß zwischen dem, beispielsweise als Stabilisierungsring ausgebildete n,Abstützelement und der zugehörigen Gegenfläche des Hohlzapfens bei entspanntem Stopfen ein Zwischenraum besteht.

Führt man einen derartig ausgebildeten Stopfen in die entsprechende Entnahmeöffnung des Behälterkörpers ein, so deformiert sich der Hohlzapfen verhältnismäßig leicht, bis er an dem Abstützelement anliegt. Eine weitere elastische Verformung des Hohlzapfens wird durch das Abstütz-zelement weitgehend verhindert. Dadruch wird eine hohe Andruckkraft des Dichtwulstes und damit eine gute Dichtigkeit bewirkt.

Obwohl es mit der in der deutschen Patentschrift 24 06 558 beschriebenen Konstruktion möglich ist, Verschlußstopfen aus Kunststoff für die Verpackung von Teststreifen einzusetzen, können derartige Verpackungen nicht in jeder Hinsicht befriedigen. Sie sind nach langer Lagerzeit häufig schwer zu öffnen. An die Toleranzen der Fertigung, sowohl der Stopfen als auch der Behälterkörper, werden hohe Anforderungen gestellt. Schon bei geringsten Abweichungen kann es vorkommen,daß derartige Verpackungen undicht werden.

Der Erfindung liegt die Aufgabe zugrunde, einen Behälter für Teststreifen zu schaffen, der die genannten hohen Anforderungen erfüllt, dennoch einfach zu handhaben und herzustellen ist.

Die Aufgabe wird bei einem Behälter der eingangs bezeichneten Art dadurch gelöst, daß das Abstützelement so ausgebildet ist, daß es den Hohlzapfen in einem gegenüber der Scheitellinie des Dichtwulstes von der Kopfplatte weg axial versetzten Bereich abstützt, während axial in Höhe des Dichtwulstes zwischen der Innenseite des Hohlzapfens und der Trockenmittelzelle auch im in die Entnahmeöffnung eingesetzten Zustand des Stopfens ein Ringspalt vorhanden ist, so daß der Hohlzapfen im Bereich des Dichtwulstes radi al nach innen elastisch deformierbar ist. Die Erfindung weicht also von der Konzeption der deutschen Patentschrift 24 06 558 grundlegend ab, indem sie das Abstützelement gerade nicht dort anordnet, wo sich der Dichtwulst befindet. Erfindungsgemäß bildet vielmehr das Abstützelement ein zum Behälterinneren hin versetztes Auflager für den näherungsweise zylinderförmig ausgebildeten Hohlzapfen Ein zweites Auflager wird dadurch gebildet, daß der Hohlzapfen an die Kopfplatte des Stopfens angegossen ist Zwischen diesen beiden Auflagern kann sich der Hohlzapfen derartig elastisch verformen, daß eine definierte, radiale Um-fangsspannung erzeugt wird, deren Spannungsab-bau bei konstanter Verformung gering bleibt. Die Spannung kann je nach Bedarf über die Wanddicke des Hohlzapfens und die Dicke des Dichtwulstes reguliert werden.

Überraschenderweise hat sich gezeigt, daß mit einer derartigen Konstruktion trotz des verhältnismäßig geringen Anpreßdruckes eine hervorragende Langzeitdichtigkeit erreicht wird. Dennoch läßt sich der Stopfen jederzeit verhältnismäßig leicht abnehmen und wieder einsetzen, ohne daß er festklemmt.

Weitere Vorteile sind darin zu sehen, daß sich der Stopfen des erfindungsgemäßen Behälters aus einem geeigneten Kunststoff, beispielsweise Polyäthylen, problemlos einteilig herstellen läßt, daß die Konstruktion platzsparend ist, so daß der Stopfen bei gegebener Trockenmittelmenge verhältnismäßig kurz ausgebildet sein kann und daß zurHerstellung des Stopfens verhältnismäßig wenig Material gebraucht wird. Diese Vorteile treffen in besonderem Maße zu, wenn das Abstützelement gemäß bevorzugter Ausführungsform als wasserdampfdurchlässige Scheibe ausgebildet ist, die zugleich die Trockenmittelzelle abdeckt. Geeignet ist insbesondere eine Kartonscheibe, wie sie üblicherweise zur Abdeckung von Trockenmittel verwendet wird.

Gemäß einer weiteren bevorzugten Ausführungsform ist die Entnahmeöffnung gegenüber dem Durchmesser der Dichtfläche zum Rand der Öffnung hin verengt ausgebildet. Im Zusammenhang mit der verhältnismäßig hohen Elastizität des erfindungsgemäß ausgebildeten Stopfens ergeben sich damit besondere Vorteile. Der Stopfen rastet hörbar und definiert in die Verschlußstellung ein. Ein versehentliches Herausrutschen des Stopfens wird auch bei großen Luftdruckschwankungen zuverlässig vermieden. Dennoch läßt sich der Behälter auch bei dieser Ausführungsform leicht öffnen und verschließen. Dies gilt insbesondere, wenn die Verengung am Rand der Öffnung als Mehrzahl von Haltenoppen ausgebildet ist, die um den Rand der Öffnung herum verteilt sind.

Die Erfindung wird im folgenden anhand von in den Figuren schematisch dargestellten Ausführungsbeispielen näher erläutert. Es zeigen:

Fig. 1 einen Querschnitt durch einen erfindungsgemäßen Behälter, wobei der Stopfen teil weise im Schnitt, teilweise in Seitenansicht dargestellt ist.

Fig. 2 einen Schnitt durch einen Behälterkörper

Fig. 3 einen Ausschnitt III aus Figur 2.

Der insgesamt mit 1 bezeichnete Teststreifenbehälter besteht aus einem kreiszylinderförmigen Behälterkörper 2 und einem Verschlußstopfen 3, welcher die Entnahmeöffnung 2a des Behälterkörpers verschließt.

Der Behälterkörper ist kreiszylinderförmig gestaltet. Das untere, in den Figuren nicht dargestellte Ende, ist geschlossen.

Der Stopfen 3 ist aus Kunststoff, beispielsweise Polyäthylen oder auch Polypropylen, im Spritzgußverfahren hergestellt. An eine Kopfplatte 4, deren umlaufender Rand wie üblich geriffelt ist, ist die kreiszylinderförmige Wand 5 der Trockenmittelzelle 6 und der ebenfalls im wesentlichen kreiszylinderförmige Hohlzapfen 7 mit dem Dichtwulst 8 angegossen. Zwischen der Außenseite der Wand 5 und der Innenseite des Zapfens 7 befindet sich ein Ringspalt 9.

Die Trockenmittelzelle 6 ist zum Behälterinneren hin mit einer wasserdampfdurchlässigen Kartonscheibe 10 abgedeckt. Die Kartonscheibe 10 verschließt nicht nur die Trockenmittelzelle, so daß das darin enthaltene Trockenmittel 11 nicht herausfallen kann, sondern sie dient gleichzeitig als Abstützelement für den Hohlzapfen 7. Zweckmäßigerweise befindet sich am Unterende des Hohlzapfens 7 eine Aufnahmenut 12, in die die Kartonscheibe 10 bei der Montage des Stopfens eingelegt wird. Sie wird dann durch Umbördeln der vorstehenden Kunststoffnase unter Temperatureinwirkung fixiert.

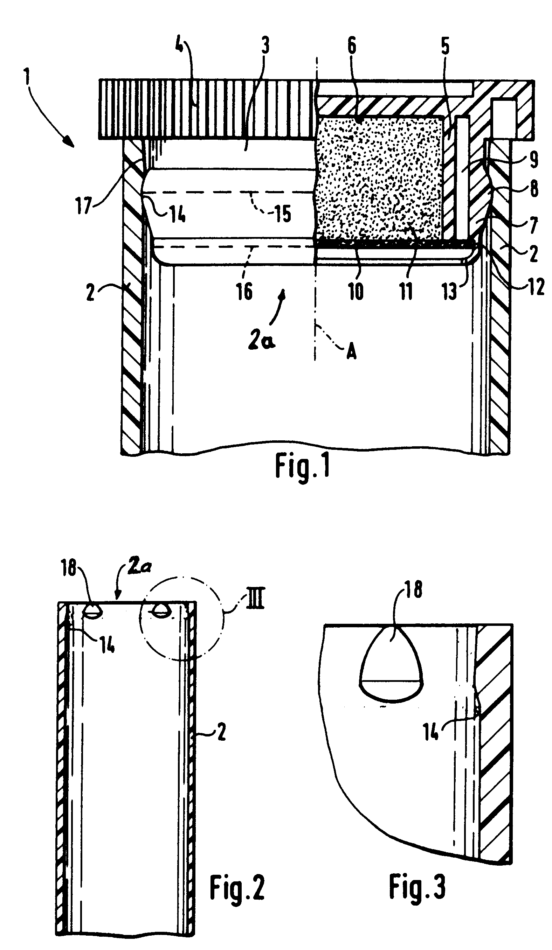

Der Behälter ist kreissymmetrisch um die Zentralachse A ausgebildet. Der Dichtwulst 8 drückt innenseitig gegen eine zur Achse A hin orientierte Dichtfläche 14 an der Innenwand des Behälterkörpers 2. Die Linie maximaler Ausdehnung des Ringwulstes 8 ist die in Figur 1 gestrichelt dargestellte Scheitellinie 15. Die Kartonscheibe 10 stützt den Hohlzapfen 7 entlang einer Stützlinie 16, die ebenfalls gestrichelt dargestellt ist.

Statt der Kartonscheibe 10 könnte auch ein anderes Stützelement verwendet werden, beispielsweise ein eingelegtes Speichenrad. Es könnte auch das untere Ende des Dichtwulstes 8 deutlich verstärkt ausgebildet sein, so daß es selbst als Stützelement wirkt. Die dargestellte Ausführungsform ist jedoch besonders einfach in der Herstellung und praktisch in der Montage.

Aus Figur 1 ist deutlich zu ersehen, daß die zylindrische Wandung des Hohlzapfens 7 mit dem Ringwulst 8 einerseits an ihrem Oberende mit der Kopfplatte 4 fest verbunden ist und andererseits sich in der Nähe ihres unteren Endes auf das Abstützelement 10 abstützt. Bevorzugt ist das Abstützelement dabei so gestaltet, daß es schon im entspannten Zustand des Stopfens den Hohlzapfen von innen berührt. Im Gegensatz dazu soll der Ringspalt 9 zwischen dem Zapfen 7 und der äußeren Begrenzung der Trockenmittelzelle 6 auch im in denBehälterkörper 2 eingesetzten Zustand des Stopfens nicht geschlossen werden.

Die erfindungsgemäße Wirkung beruht nun wesentlich darauf, daß der Hohlzapfen 7 im Bereich des Ringwulstes 8 nach radial innen elastisch nachgeben kann, dennoch aber durch die beidseitige Lagerung zuverlässig nach außen gedrückt wird. Dabei spielen auch die Abmessungen des Hohlzapfens eine erhebliche Rolle. Bei einer bevorzugten Ausführungsform wird für einen Stopfen mit einem Außendurchmesser von ca. 25 - 30 mm ein Hohlzapfen verwendet, dessen Wandung im zylindrischen Bereich ca. 1 mm stark ist, wobei der Wulst im entspannten Zustand um etwa 1 mm aus der Zylinderfläche hervorragt.

Für die Funktion des Stopfens ist es günstig, wenn die Scheitellinie 15 des Dichtwulstes einen Mindestabstand in axialer Richtung sowohl von der Kopfplatte 4 als auch von der Stelle hat, an der sich das Abstützelement abstützt (im dargestellten Fall der Stützlinie 16). Bevorzugt beträgt dieser Mindestabstand jeweils mindestens 2 mm, besonders bevorzugt mindestens 3 mm.

Der Behälterkörper 2 ist oberhalb der Dichtfläche 14, also zum Rand der Entnahmeöffnung 2a hin, verengt. Bei der in Figur 1 dargestellten Ausführungsform wird die Verengung durch einen Ringwulst 17 gebildet, d.h. der obere Rand des Behälterkörpers 2 ist durchgehend nach innen verdickt. Dadurch erhält das offene Ende des Behälterkörpers 2 zusätzliche Stabilität und der Stopfen 3 rastet definiert in den Behälterkörper 2 ein.

In den Figuren 2 und 3 ist eine Ausführungsform eines Behälterkörpers 2 dargestellt, bei der die Verengung in Form einer Mehrzahl von Haltenoppen 18 realisiert ist, die auf der Innenseite der Wand des Behälterkörpers oberhalb der Dichtfläche 14 ausgebildet sind. Die Haltenoppen 18 laufen nach oben und unten flach zu. Sie erlauben ein besonders einfaches Öffnen und Schließen des Behälters.

## Patentansprüche

1. Behälter für Teststreifen zur Analyse von Körperflüssigkeiten umfassend
   einen Behälterkörper (2) mit einer kreisförmigen Entnahmeöffnung (2a) und einer zur Achse (A) der Öffnung weisenden Dichtfläche (14) und
   einen Stopfen (3) zum Verschließen der Entnahmeöffnung (2a) , welcher eine Kopfplatte (4), einen daran angegossenen Hohlzapfen (7) mit einem nach außen weisenden Dichtwulst (8), eine Trockenmittelzelle (6) innerhalb des Hohlzapfens und ein Abstützelement (10), durch das der Zapfen radial nach innen abgestützt wird, aufweist,
   **dadurch gekennzeichnet, daß**
   das Abstützelement so ausgebildet ist, daß es den Hohlzapfen in einem gegenüber der Scheitellinie (15) des Dichtwulstes (8) von der Kopfplatte (4) weg axial versetzten Bereich abstützt, während axial in Höhe des Dichtwulstes (8) zwischen der Innenseite des Hohlzapfens und der Trockenmittelzelle auch im in die Entnahmeöffnung eingesetzten Zustand des Stopfens (3) ein Ringspalt (9) vorhanden ist, so daß der Hohlzapfen (7) im Bereich des Dichtwulstes (8) radial nach innen elastisch deformierbar ist.

2. Behälter nach Anspruch 1, **dadurch gekennzeichnet, daß**
   das Abstützelement eine wasserdampfdurchlässige Scheibe (10) ist, die zugleich die Trockenmittelzelle (6) abdeckt.

3. Behälter nach Anspruch 2, **dadurch gekennzeichnet, daß**
   die Scheibe (10) am von der Kopfplatte (4) abgewandten Ende des Hohlzapfens (7) eingebördelt ist.

4. Behälter nach Anspruch 1, **dadurch gekennzeichnet, daß**
   die Stützstelle des Abstützelementes (10) gegenüber der Scheitellinie (15) des Dichtwulstes (8) um mindestens 2 mm, bevorzugt mindestens 3 mm axial versetzt ist.

5. Behälter nach Anspruch 1, **dadurch gekennzeichnet, daß**
   die Entnahmeöffnung (2a) gegenüber dem Durchmesser der Dichtfläche (14) zum Rand der Öffnung hin auf wenigstens einem Teil des Umfangs verengt ist.

6. Behälter nach Anspruch 5, **dadurch gekennzeichnet, daß**
   die Verengung als durchgehender Ringwulst (17) ausgebildet ist.

7. Behälter nach Anspruch 5, **dadurch gekennzeichnet, daß**
   die Verengung als Mehrzahl von Haltenoppen (18) ausgebildet ist.

## Claims

1. Container for test strips for the analysis of body fluids, comprising a container body (2) with a circular removal opening (2a), a sealing surface (14) facing the axis (A) of the opening and a stopper (3) for the closure of the removal opening (2a) which has a cover plate (4), a hollow plug (7) attached thereto with an outwardly facing sealing beading (8), a drying agent cell (6) within the hollow plug and a support element (10) by means of which the plug is supported radially inwardly, characterised in that the support element is so constructed that it abuts the hollow plug in a region axially displaced away from the cover plate (4) with regard to the zenithal line (15) of the sealing beading (8), whereas axially, at the height of the sealing beading (8), between the inner side of the hollow plug and the drying agent cell, even when the stopper (3) is in its inserted position, an annular gap (9) is present so that the hollow plug (7) is radially inwardly elastically deformable in the region of the sealing beading (8).

**2.** Container according to claim 1, characterised in that the support element is a water vapour-permeable disc (10) which simultaneously covers the drying agent cell (6).

**3.** Container according to claim 2, characterised in that the disc (10) is flanged in on the end of the hollow plug (7) facing away from the cover plate (4).

**4.** Container according to claim 1, characterised in that the point of abutment of the support element (10) is axially displaced with regard to the zenithal line (15) of the sealing beading (8) by at least 2 mm, preferably at least 3 mm.

**5.** Container according to claim 1, characterised in that the removal opening (2a) is narrowed on at least a part of its circumference towards the edge of the opening with regard to the diameter of the sealing surface (14).

**6.** Container according to claim 5, characterised in that the narrowing is constructed as a continuous annular beading (17).

**7.** Container according to claim 5, characterised in that the narrowing is constructed as a plurality of holding knops (18).

**Revendications**

**1.** Récipient pour bande de test destinée à analyser des fluides corporels, comprenant
un récipient (2) muni d'un orifice de prélèvement (2a) circulaire et d'une surface d'étanchéité (14) tournée vers l'axe (A) de l'orifice, et un bouchon (3) pour obturer l'orifice de prélèvement (2a), qui présente une plaque de recouvrement (4), un tourillon creux (7) raccordé à cette dernière, avec un bourrelet d'étanchéité (8) tourné vers l'extérieur, une chambre (6) pour agent dessicant à l'intérieur du tourillon creux et un élément d'appui (10) qui assure le maintien radial du tourillon,
caractérisé en ce que l'élément d'appui est conçu de façon à maintenir le tourillon creux dans un domaine axialement décalé de la plaque de recouvrement (4) par rapport au sommet (15) du bourrelet d'étanchéité (8), tandis qu'une fente annulaire (9) est prévue axialement à la hauteur du bourrelet d'étanchéité (8) entre la face intérieure du tourillon creux et la chambre contenant l'agent dessicant, également dans l'état où le bouchon (3) est introduit dans l'orifice de prélèvement, de sorte que le tourillon creux (7) peut subir une déformation élastique radialement vers l'intérieur au voisinage du bourrelet d'étanchéité (8).

**2.** Récipient selon la revendication 1, caractérisé en ce que l'élément d'appui est un disque (10) perméable à la vapeur d'eau, qui recouvre en même temps la chambre (6) contenant l'agent dessicant.

**3.** Récipient selon la revendication 2, caractérisé en ce que le disque (10) est serti à l'extrémité du tourillon creux (7) opposée à la plaque de recouvrement (4).

**4.** Récipient selon la revendication 1, caractérisé en ce que le point d'appui de l'élément d'appui (10) est décalé axialement par rapport au sommet (15) du bourrelet d'étanchéité (8) d'au moins 2 mm, de préférence d'au moins 3 mm.

**5.** Récipient selon la revendication 1, caractérisé en ce que l'orifice de prélèvement (2a) se rétrécit sur au moins une partie de sa périphérie par rapport au diamètre de la surface d'étanchéité (14), vers le bord de l'orifice.

**6.** Récipient selon la revendication 5, caractérisé en ce que le rétrécissement est conçu sous forme de bourrelet annulaire (17) continu.

**7.** Récipient selon la revendication 5, caractérisé en ce que le rétrécissement est conçu sous forme d'une pluralité de boutons de fixation (18).

Fig.1

Fig.2

Fig.3